# EUROPEAN PATENT APPLICATION

(11) **EP 3 363 517 A1**
(43) Date of publication of application: **22.08.2018**
(21) Application number: 17156600.3
(22) Date of filing: 17.02.2017
(51) Int. Cl.: B01D 19/00, C07K 1/36, C12M 1/00

(54) **DEGASSING IN METHODS FOR CONTINUOUS PRODUCTION OF A HEALTHCARE PRODUCT**

(71) Applicant: Bayer Healthcare LLC, Whippany, NJ 07981 (US)
(72) Inventor: Schwan, Peter, 51373 Leverkusen (DE); Lobedann, Martin, 51069 Köln (DE)
(74) Representative: BIP Patents

(57) **Abstract**

Described herein are the use of at least one dense membrane and/or at least one nanoporous membrane in a method for the continuous, pathogen-reduced production and/or processing of a healthcare product. For example the at least one dense membrane and/or the at least one nanoporous membrane is used as degasser and/or as de-bubbler and/or as pathogen barrier

## Description

Conventionally, healthcare products such as biotechnological proteins are purified in batches. Thus, the individual production cycles are handled batchwise and discontinuously with the product being removed completely at one time point after completion of a production cycle. For a fresh production cycle a fresh batch must then be started. Since this batchwise production is time-consuming, difficult to scale up and expensive, new ways for the manufacture of healthcare products such as biotechnological proteins are explored. Therefore, continuous processing for the production of therapeutic proteins gains more and more importance and first solutions for realization of truly continuous systems are emerging

In both conventional batchwise production processes as well as continuous production processes trapped gas, especially air, forming gas bubbles can potentially disrupt the production process to a significant extend. This is the case as the gas bubbles can partly or completely prevent the fluid stream - comprising the desired product - from passing a unit operation such as filtration and/or can prevent the normal conduction of a given unit operation such as a chromatography to a significant extend. In addition, gas bubbles can cause errors e.g. to sensors and chromatography columns mainly by letting components dry and gas bubbles present inside a sample can lead to pipetting and sampling errors. Thus, in conventional batchwise production processes bubble traps - such as the Biorad bubble trap-are used to dissolve gas bubbles. However, because the mobile phase level of the bubble trap requires constant monitoring, automatic control and operation of such a bubble trap are prone to errors, complicated and difficult to realize.

As an alternative to dissolving gas bubbles in a fluid, the gas can also be removed via degassing of the fluid. A method for degassing a fluid stream of a continuous production process is described in EP3015542 A1. Specifically, EP3015542 A1 describes the use of a hydrophobic microfiltration membrane - e.g. a Membrana micro-module - operated with a vacuum. Compared to a bubble trap such a hydrophobic microfiltration membrane has the advantages that it is sterilizable and can be used in a continuous fashion. Moreover, since the hydrophobic microfiltration membrane does not comprise a mobile phase and hence facilitates control by a process control system.

However, during operation of such hydrophobic microfiltration membranes liquid breakthrough onto the vacuum side can occasionally occur. This can potentially destroy the vacuum system and hence the sterility or at least the pathogen-reduced state of the process is at least in theory put at risk. In a process, which should ideally meet regulatory requirements set by healthcare authorities, this risk has to be minimized.

Therefore, there is a need for an optimized solution for minimizing the risk that dissolving of gas bubbles and/or degassing of a fluid stream required in continuous, pathogen-reduced methods for production and/or processing of healthcare products could potentially affect the the pathogen-reduced state of said process.

For the first time it was surprisingly found that this objective can be met by using a dense membrane as pathogen barrier.

Thus, in a first aspect the present invention relates to the use of a dense membrane as pathogen barrier.

As used herein the term "dense membrane" refers to a membrane comprising at least one separation layer that is characterized by having no pores that would permit a convective mass transfer of liquid through the membrane. In other words, a dense membrane comprises at least one separation layer, which does not permit mass transfer via bulk motion of a fluid.

As used herein the term "pathogen barrier" refers to a material, e.g. a membrane, exclusively with pores having a size of between ≥ 0.01 µm and ≤ 0.2µm. Since all pores are smaller than ≤0.2µm the pathogen barrier prevents microorganisms such as bacteria, archaea and protozoa from passing to an extent that allows for a bioburden controlled continuous process for more than 24h.

This use of a dense membrane as a pathogen barrier is advantageous since no liquid breakthrough onto the vacuum side can occur during operation, since the dense membrane does not have pores that allow fluid to pass. Hence, the risk that process fluid leaks out at a debubbling and/or degassing point e.g. into a vacuum system, which is difficult if not impossible to sanitize during production, is minimized or avoided all together

As used herein the term "pathogen-reduced" is used interchangeable with "low-bioburden","microbe-reduced" and "germ-reduced" and refers to a state of reduced pathogenic count, i.e. a pathogenic count per area or volume unit of close to zero that is achievable by means of a suitable germ-reducing method, wherein this germ-reducing method can be selected from gamma irradiation, beta irradiation, autoclaving, Ethylene Oxide (ETO) treatment, Ozone treatment, "Steam-In-Place" (SIP) and/or Heat in Place treatment or treatment with sanitization agent like 1 M NaOH.

Moreover, it was surprisingly found for the first time that at least one dense membrane and/or at least one nanoporous membrane can be used in a method for the continuous, pathogen-reduced production and/or processing of a healthcare product.

As used herein the term "continuous" refers to a method for carrying out at least two method steps and/or unit operations in series in which the outlet fluid stream (fluid flow) of an upstream step is transported to a downstream step. The downstream step begins processing the fluid flow before the upstream step is completed. Accordingly, continuous transport or transfer of a fluid flow from an upstream unit to a downstream unit means that the downstream unit is already in operation before the upstream is shut down, i.e. that two units connected in series simultaneously process the fluid flow that is flowing through them.

As used herein the term "fluid stream" or "fluid flow" refers to a continuous flow of liquid and/or gas. The fluid stream or fluid flow can comprise a product.

As used herein the term "healthcare product" refers to products used to diagnose, treat or care for patients such as to intermediate or active ingredients produced by pharmaceutical industry.

While it is possible to use a combination of any number of dense membranes with any number of nanoporous or even hydrophobic microporous membranes in a method for the continuous, pathogen-reduced production and/or processing of a healthcare product, a person skilled in the art can identify situations where it might be suitable to employ only dense membranes or only nanoporous membranes or only hydrophobic microporous membranes in a method for the continuous, pathogen-reduced production and/or processing of a healthcare product.

In one embodiment of the use of a dense membrane and/or the nanoporous membrane as described herein, the healthcare product is or comprises at least one component selected from the group consisting of a peptide, protein, a small molecule drug, a nucleic acid.

As used herein the term "peptide" refers to a polymer of amino acids of relatively short length (e.g. less than 50 amino acids). The polymer may be linear or branched, it may comprise modified amino acids, and it may be interrupted by non-amino acids. The term also encompasses an amino acid polymer that has been modified; for example, by disulfide bond formation, glycosylation, lipidation, acetylation, phosphorylation, or any other manipulation, such as conjugation with a labeling component, such as but not limited to, fluorescent markers, particles, biotin, beads, proteins, radioactive labels, chemiluminescent tags, bioluminescent labels, and the like.

As used herein the term "protein" refers to a polypeptide of amino acids. The term encompasses proteins that may be full-length, wild-type, or fragments thereof. The protein may be human, non- human, and an artificial or chemical mimetic of a corresponding naturally occurring amino acid, as well as to naturally occurring amino acid polymers and non- naturally occurring amino acid polymer.

Preferably the protein is a therapeutic protein.

As used herein the term "therapeutic protein" refers to a protein that can be administered to an organism to elicit a biological or medical response of a tissue, an organ or a system of said organism.

Even more preferably the protein is an antibody.

The term "antibody" as used herein refers to a binding molecule such as an immunoglobulin or immunologically active portion of an immunoglobulin, i.e., a molecule that contains an antigen-binding site.

As used herein the term "small molecule drug" refers to a low molecular weight (<900 daltons) compound that may help regulate a biological process.

As used herein, the term "nucleic acid" refers to deoxyribonucleotides or ribonucleotides and polymers thereof in either single- or double-stranded form. Unless specifically limited, the terms encompass nucleic acids containing analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g. degenerate codon substitutions) and complementary sequences as well as the sequence explicitly indicated.

The finding that at least one dense membrane and/or at least one nanoporous membrane can be used in a method for the continuous, pathogen-reduced production and/or processing of a healthcare product was surprising as until now no membranes at all or in exceptional cases hydrophobic microporous membranes were used in continuous, pathogen-reduced production processes of healthcare products.

Moreover, porous membranes differ from dense membranes used as described herein in that the porous membranes comprise least one separation layer with pores permitting a convective mass transfer of liquid through the membrane, i.e. allow a wetting of the pores with a fluid.

As used herein the term "nanoporous" refers to a material with pores having a size of between ≥ 0.01µm and ≤ 0.2µm.

As used herein the term "microporous " refers to a material comprising pores with diameters >0.3 µm and < 2 µm and/or to a material comprising cavities with sizes less than 30 µm. An example of microporous membranes are the hydrophobic microfiltration membranes used in EP3015542 A1.

In one embodiment of the use of a dense membrane and/or a nanoporous membrane as described herein, the dense membrane and/or the nanoporous membrane is used as degasser and as pathogen barrier.

As used herein the term "degasser" or "degassing" refers to a device or a process that removes gas bubbles or dissolved gas from a liquid. In other words, immediately following degassing, less gas is present in said fluid.

The finding that a dense membrane can be used as degasser is especially surprising since the gas bubbles have to wet the membrane prior to degassing and said wetting is in theory prevented by the surface tension of the tight membrane, which the gas bubbles have to overcome. Thus, in theory dense membranes cannot be used as degasser.

Likewise nanoporous membranes have a comparatively high surface tension, hence the finding that nanoporous membranes can be used as degasser is also surprising.

In one embodiment of the use of a dense membrane as described herein the dense membrane is gas-permeable but not air-permeable.

In a preferred embodiment of the use of a dense membrane described herein, the membrane comprises ultra/superhydrobic polyolefin.

In an especially preferred embodiment the dense membrane is a superphobic® 1x3 membrane contractor G681 W membrane module.

In one embodiment of the use of a dense membrane and/or a nanoporous membrane as described herein, the dense membrane and/or the nanoporous membrane is situated in a fluid stream and the fluid stream is passed top to bottom over the dense membrane.

This passing from top to bottom is advantageous as it causes gas bubbles to rise countercurrently to the fluid stream due to gravity and hence upstream, whereas the fluid stream in a continuous production process is typically led downstream to a subsequent unit operation. Hence, a dense membrane and/or a nanoporous membrane situated in this fashion in a fluid stream leads to a separation of gas bubbles from the fluid stream due to gravity. Therefore, the top to bottom passing facilitates debubbling of the fluid stream. In other words, it was surprisingly found that the top-to bottom passing of the fluid stream eliminates the risk that gas bubbles can pass freely through a dense membrane or a nanoporous membrane. In theory this risk is due to the fact that a dense membrane or a nanoporous membrane has a low specific degassing rate. Thus, in order to overcome this risk of freely passing gas bubbles very large dense membrane modules or very large nanoporous membranes would have to be employed, if the fluid stream is passed bottom to top over the dense membrane or the nanoporous membrane. However, via passing the fluid stream top to bottom over the dense membrane or the nanoporous membrane a controlled debubbling of the fluid stream is possible.

As used herein the term "debubbling" or "debubbler" refers to a device or a process that prevents gas bubbles from flowing with a fluid stream. In other words, immediately following debubbling, less gas bubbles are present in said fluid stream, but the gas content of the whole of said fluid stream is not altered.

Thus, a dense membrane and/or a nanoporous membrane can act as debubbler, if a fluid stream is passed over it top to bottom, because gravity causes gas bubbles to rise countercurrently to the fluid stream and hence separates gas bubbles from the fluid stream as described above. In other words, at the phase boundary interface upstream of the dense membrane and/or the nanoporous membrane gas bubbles are separated from the fluid stream. Hence the dense membrane and/or the nanoporous membrane cts as gas liquid separator physically separating gas bubbles from the downwardly flowing fluid stream.

Preferably the dense membranes described herein act as degasser, as debubbler and as pathogen barrier. This embodiment has the advantage that it represents an optimized solution for debubbling and/or degassing of a fluid stream especially under the sterile or pathogen-reduced conditions required in continuous, pathogen-reduced methods for production and/or processing of healthcare products

Preferably the nanoporous membranes described herein act as degasser, as debubbler, as pathogen barrier and as gas-liquid separator.

In one embodiment of the use of a dense membrane and/or a nanoporous membrane as described herein the dense membrane and/or the nanoporous membrane is operated using a vacuum.

If the dense and/or the nanoporous membrane is comprised in a membrane module operated using a vacuum, this membrane module preferably comprises a gas phase and a liquid phase formed by the fluid of the fluid stream which usually comprises the product. In other words, gas can pass the dense and/or the nanoporous membrane in the direction of the vacuum both from the gas phase and from the liquid phase. In such a setting using a vacuum has the effect that in said membrane module comprising a dense and/or a nanoporous membrane the gas removal rate is larger in the gas phase than in the liquid phase, where additional mass transfer resistances in the liquid membrane barrier can occur. However, despite said resistance gas removal in the direction of the vacuum will also occur from the liquid phase due to the difference of partial pressures of the dissolved gases in the liquid phase and the vacuum side of the dense and/or the nanoporous membrane. Overall, gas in such a setting may pass the dense and/or the nanoporous membrane in the direction of the vacuum from the gas phase and from the liquid phase as well as from the liquid phase to the gas phase on the non-vacuum side of the dense and/or the nanoporous membrane.

In a preferred embodiment of operating the dense membrane and/or the nanoporous membrane using a vacuum the dense membrane and/or the nanoporous membrane is operated using a vacuum pump.

In one embodiment of the use of a dense membrane and/or a nanoporous membrane as described herein the dense membrane and/or the nanoporous membrane is operated using a vacuum pump and said pump is protected using a liquid trap.

An example of a liquid trap is a glass bottle having a removable cap. The vacuum tube system can be connected through the cap, i.e. via two tubes. As the vacuum is applied through one tube, it exhausted the air in the bottle and induced a vacuum in the other tube. Obviously, any water which might accidentally be carried over through the one tube connected to the process system would be trapped in the glass bottle and not carried over into the other tube or the pump.

In one embodiment of the use of a dense membrane and/or a nanoporous membrane as described herein the dense membrane is controlled by a process control system.

Using a process control system has the effect that the method for the continuous, pathogen-reduced production and/or processing of a healthcare product can be automated. Automation in turn facilitates set-up of an efficient, safe, reliable, standardized production process yielding a high quality product.

As used herein the term "process control" refers to a system and the devices of that system that monitor the manufacturing environment and electronically control the process or manufacturing flow based on the various set-points given by the user.

Such a process control system can, *inter alia,* monitor the performance data such as the pump rate of the vacuum pump operating the dense membrane. Deviations in said performance data can indicate a leakage and hence can allow an early shut down of the system in order to minimize the contamination risk. For example the rotational speed of the vacuum pump is monitored and the pump is set to deliver 25 mbar. In case of a leakage the rotational speed will increase, since the pump has to pump more in order to deliver the set 25 mbar.

In one embodiment of the use of a dense membrane and/or a nanoporous membrane as described herein the dense membrane and/or the nanoporous membrane is situated in a fluid stream, which passes at least one unit operation selected from the group comprising:
- a cell separator
- an ultrafiltration unit for concentration
- a recirculation loop
- a unit for buffer or medium exchange preferably with concentration, e.g. an ultrafiltration
- a bioburden reduction preferably with sterile filters
- a capture chromatography
- a virus inactivation, e.g. a coiled flow inverter i.e. a residence time module
- a chromatographic intermediate and fine purification, e.g. ion exchange, mixed mode, hydrophobic interaction, SEC chromatography
- a homogenization loop
- a viral filtration
- a flow cell for process analytics such as pH, conductivity, flow meter,
- a sample port for in process samples

In one embodiment of the use of a dense membrane and/or the nanoporous membrane as described herein the dense membrane and/or the nanoporous membrane is situated in a fluid stream, which is selected from the group comprising: an auxiliary fluid, such as calibration buffer, cleaning solutions, pH and conductivity agent, excipiens or a fluid stream comprising a product.

As used herein the term "unit" or "unit operation" refers to a device that performs one process step in a production process of a healthcare product and to the process which that specific device performs. In other words, in order to provide the final healthcare product several unit operations will have to be passed by a fluid stream comprising the healthcare product until the product has the desired characteristics and/or the desired purity.

In a preferred embodiment of the use of a dense membrane and/or a nanoporous membrane as described herein, the continuous, pathogen-reduced production process of a healthcare product is a continuous, pathogen-reduced production of a therapeutic protein e.g. an antibody.

In a preferred embodiment of the use of a dense membrane and/or the nanoporous membrane as described herein the continuous, pathogen-reduced production process of a healthcare product uses disposable articles.

As used herein the term "disposable articles" means that the respective components coming into contact with the fluid stream, particularly equipment, containers, filters, and connecting elements, are suitable for one-time use followed by disposal, wherein these containers can be made of both plastic and metal. Within the scope of the present invention, the term also comprises disposable articles such as those made of steel that are only used once in the process according to the invention and not used again in the process. These disposable articles, for example those made of steel, are then also designated within the scope of the invention as objects "used as disposable articles." Such used disposable articles can then also be designated in the process according to the invention as "disposable" or "single-use" articles ("SU technology"). In this way, the pathogen-reduced status of the process and modular system according to the invention is improved even more.

It was surprisingly found that employing disposable tubes/disposable tubing, especially weldable tubing, requires degassing and/or debubbling of the fluid stream. Without wishing to be bound by theory this finding is thought to be due to air entering the weldable tubing at a higher and/or faster rate than anticipated, with the state of the art being silent towards this insight.

As used herein the term "weldable tubing" refers to tubes and tubings manufactured from plastic e.g. comprising silicone. Examples of weldable tubings are silicone like tubings as well as tubings comprising silicon compounds e.g. Pharmed BPT, Cflex Sanipure tubings and PVC tubings.

Hence using dense membranes and/or nanoporous membranes is especially advantageous in a continuous, pathogen-reduced production process of a healthcare product employing disposable articles.

In a preferred embodiment of the use of a dense membrane and/or a nanoporous membranes as described herein the continuous, pathogen-reduced production process of a healthcare product is modular.

As used herein the term "modular" means that the individual unit operations can be carried out in separate interconnected modules, wherein the modules are preconfigured, germ-reduced, and closed, and can be interconnected in various combinations.

As used herein the term "closed" means that the method described is operated in such a way that the fluid stream is not exposed to the room environment. Materials, objects, buffers, and the like can be added from outside, wherein, however, this addition takes place in such a way that exposure of the fluid stream to the room environment is avoided.

As used herein the term "closed" refers to both "functionally closed" as well as "closed".

In detail, a closed production plant (process system) is designed and operated such that the product is never exposed to the surrounding environment. Additions to and draws from closed systems must be performed in a completely closed fashion. Sterile filters may be used to provide effective barriers from contaminants in the environment. The term "functionally closed" refers to a process that may be opened but is "rendered closed" by a cleaning, sanitization and/or sterilization that is appropriate or consistent with the process requirements, whether sterile, aseptic or low bioburden/low-pathogen. These systems shall remain closed during production within the system. Examples include process vessels that may be CIP'd and SIP'd between uses. Non-sterile systems such as chromatography or some filtration systems may also be rendered closed in low bioburden/low-pathogen operations if appropriate measures are taken during the particular system setup.

In one embodiment of the use of a dense membrane and/or a nanoporous membranes as described herein the dense membrane and/or the nanoporous membranes is situated in a fluid stream before said fluid stream enters a unit operation and/or passes a validation point selected from the group comprising a cell separator, a chromatography, a sampling location, a unit for concentration, a diafiltration, a dialysis, a filtration, a recirculation loop, a unit for buffer or medium exchange preferably with concentration, e.g. an ultrafiltration, a virus inactivation unit, e.g. a coiled flow inverter i.e. a residence time module and/or a homogenization loop.

Conventionally, therapeutic proteins such as antibodies are purified in batches. The means that the individual production cycles are handled batchwise and discontinuously with the product being removed as a whole after completion of a production cycle. For a fresh production cycle, a fresh batch must then be started.

In such batchwise processes the fluid stream comprising the desired product is degassed before it enters a chromatographic device.

Now it was surprisingly found that in a continuous method/process for the production of a healthcare product it is advantageous that the fluid stream comprising the desired product is debubbled not only before it enters a chromatographic device but more frequently. Without wishing to be bound by this theory it is currently believed that said need to debubble more frequently - apart from the above mentioned finding that air enters disposable tubing faster/at a higher rate than expected - originates from aired storage bags used in the continuous method/process for the production of healthcare product to account for the varying speed with which different unit operations process the fluid stream. This saturation can lead to the gas forming bubbles which can in turn cause the precipitation of the healthcare product. Moreover, bubble formation can decrease the efficiency of the unit operation in question and impede or change the continuous flow of the fluid stream which can alter the residence time behavior of a fluid stream comprising a desired product. This alteration can interfere with representative sampling. Thus bubble formation is especially critical before and/or in unit operations comprising recirculation circuits e.g. ultrafiltration, filtration, residence time modules, homogenization steps and hollow fibre modules.

Typically, several dense membranes and/or nanoporous membranes will be employed in a continuous, pathogen-reduced production process of a healthcare product. Instead of employing only dense membranes and/or nanoporous membranes at all critical points it might be reasonable to employ microporous hydrophobic membranes with a bubble-point > 3 bar or hydrophobic ultrafiltration membranes with a bubble-point > 3 bar. In other words, the continuous method/process for the production of a healthcare product described herein can utilize a combination of dense membranes and/or nanoporous and hydrophobic microporous membranes.

However, a person skilled in the art can identify situations where it might be suitable to employ only dense membranes or only nanoporous membranes or only hydrophobic microporous membranes in a continuous, pathogen-reduced production process of a healthcare product.

As used herein the term "bubble point" refers to the pressure at which a continuous stream of bubbles is initially seen downstream of a wetted filter under gas pressure. It is conventionally measured using a bubble point test. To perform a Bubble Point Test, gas is applied to one side of a wetted filter, with the tubing downstream of the filter submerged in a bucket of water. The filter must be wetted uniformly such that water fills all the voids within the filter media. When gas pressure is applied to one side of the membrane, the test gas will dissolve into the water, to an extent determined by the solubility of the gas in water. Downstream of the filter, the pressure is lower. Therefore the gas in the water on the downstream side is driven out of solution. As the applied upstream gas pressure is increased, the diffusive flow downstream increases proportionally. At some point, the pressure becomes great enough to expel the water from one or more passageways establishing a path for the bulk flow of air. As a result, a steady stream of bubbles should be seen exiting the submerged tubing. The pressure at which this steady stream is noticed is referred to as the bubble point.

In another aspect the invention relates to a method for the continuous, pathogen-reduced, modular production and/or processing of a healthcare product, wherein at least one dense membrane and/or at least one nanoporous membrane is employed for degassing and/or debubbling a fluid stream, wherein the fluid stream is passed top to bottom over the dense membrane.

This method is advantageous as it prevents gas bubbles from entering a unit operation, a pathogen barrier to the environment is maintained and the risk of an accidental leakage, that can lead to microbial back growth, is minimized.

In yet another aspect the invention relates to a unit operation for the continuous, pathogen-reduced, modular production and/or processing of a healthcare product comprising at least one dense membrane and/or at least one nanoporous membrane.

Said unit operation is preferably selected from the group comprising:
- a cell separator
- a unit for buffer or medium exchange preferably with concentration, e.g. an ultrafiltration
- a bioburden reduction preferably with sterile filters
- a capture chromatography
- a virus inactivation, e.g. a coiled flow inverter, i.e. a residence time modile
- a chromatographic intermediate and fine purification, e.g. an anion exchange chromatography
- a bioburden reduction e.g. with sterile filters
- a homogenization loop
- a viral filtration

### FIGURES:

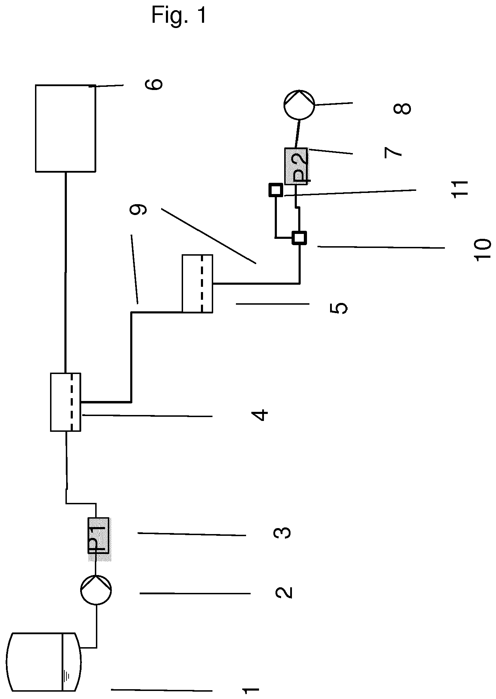

Fig. 1 shows a schematic drawing of a pathogen-reduced method for operating the hydrophobic microfiltration membranes known in the state of the art for use in continuous pathogen-reduced methods for the production of therapeutic proteins.

A gas saturated fluid is pumped from a reservoir (1) by pump (2), which has a pressure sensor (3) and is degassed using a the hydrophobic microfiltration membrane (4). The fluid flows into a unit operation (6), which is sensitive to the presence of gas bubbles. A vacuum pump (8) is used for the degassing at the hydrophobic microfiltration membrane (4). A sterile hydrophobic microfiltration membrane (5) ensures the pathogen-reduced state. During operation the pressure generated by the vacuum pump (8), which is measured at sensor (7) is not to exceed the partial pressure of water, as otherwise a membrane distillation of water will occur. Membrane (4) and membrane (5) are connected via vacuum resistant silicone tubing. During operation pressure sensor (3) has to be monitored to ensure that the bubble-point of hydrophobic microfiltration membrane (4) is not exceeded as otherwise the pores of the filter would be wetted, which could potentially lead to microbial back growth and hence could destroy the pathogen-reduced state. If a filter in the vacuum area has to be replaced while ensuring the pathogen-reduced state the following procedure has to be carried out. Since the tubing (9) cannot be welded, aseptic connectors (10), (11) have to be employed. In this example the aseptic connector (11) acts as replacement connection on the vacuum side for the replacement assembly consisting of hydrophobic microfiltration membranes(4) and (5) as well as the tubing (9).

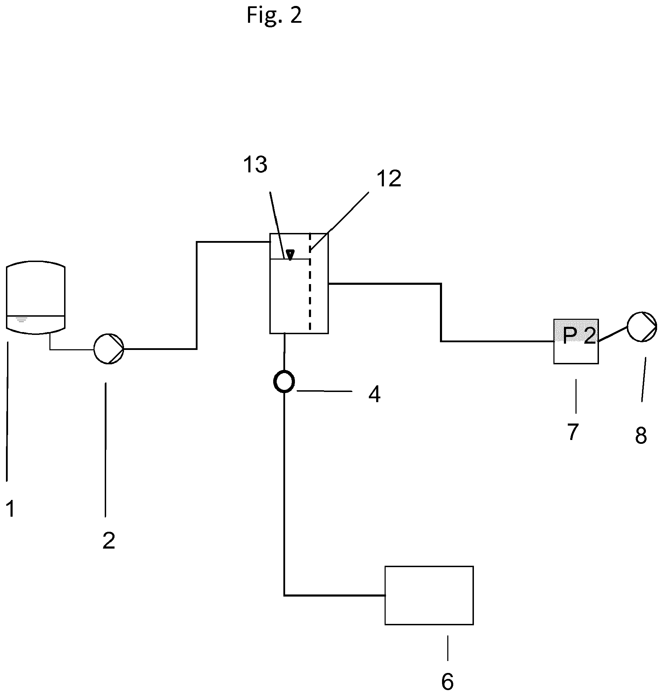

FIG. 2 shows a schematic drawing of a dense membrane used as debubbler, degasser and as pathogen barrier in a continuous pathogen-reduced method for the production of therapeutic proteins.

A gas saturated fluid stream is pumped from a reservoir (1) by pump (2) and is degassed and debubbled using a membrane module comprising a dense membrane (12). The degassed and debubbled fluid flows downstream into a unit operation (6), which is sensitive to the presence of gas bubbles. A vacuum pump (8) is connected to the membrane module comprising the dense membrane (12). The dense membrane (12) also acts as pathogen barrier. During operation the pressure generated by the vacuum pump (8), which is measured at sensor (7), should not exceed the partial pressure of water, as otherwise a membrane distillation of water will occur. Moreover, during operation the fluid stream comprising the product passes through the membrane module comprising the dense membrane (12) from top to bottom resulting in the formation of a phase boundary interface (13) between the fluid stream (liquid) and the gas phase in the membrane module comprising a dense membrane (12). Hence the membrane module comprising a dense membrane (12) comprises a predetermined amount of the fluid stream (liquid) and as well as a predetermined volume of gas. The passing from top to bottom ensures that gas bubbles will rise by gravity countercurrently to the fluid stream away from the fluid stream passing downstream to unit operation (6). Due to the vacuum generated by vacuum pump (8) this separated gas permeates through the dense membrane resulting in a controlled ratio of the gas phase and the liquid phase in the membrane module comprising a dense membrane (12), i.e. the height of the gas/liquid is controlled. In addition to the debubbling at the phase boundary interphase and the transfer of gas through the membrane in the direction of the vacuum, the fluid stream is also degassed in the liquid (fluid stream) below the phase boundary interface (13) due to the difference of partial pressures of the dissolved gases in the liquid phase and the vacuum side of the dense membrane.

Furthermore the dense membrane in the membrane module comprising a dense membrane (12) also acts as pathogen barrier.

To ensure that no gas bubbles are present in the fluid stream leaving the membrane module comprising a dense membrane (12), a gas bubble detector (4) is be employed. These are known to those skilled in the art e.g. from usage in chromatography devices. One example of such a gas bubble detector is an ultrasonic sensor, which can be monitored using a process control system. If the process control system detects an irregularity e.g. the rotational speed of the pump is higher than it should be or bubbles are measured by detector (4), pump (2) can immediately be switched off or the fluid stream can be directed to a waste disposal site via an additional tubing (not depicted). If the performance of the dense membrane (12) decreases it can be replaced via sterile welding.

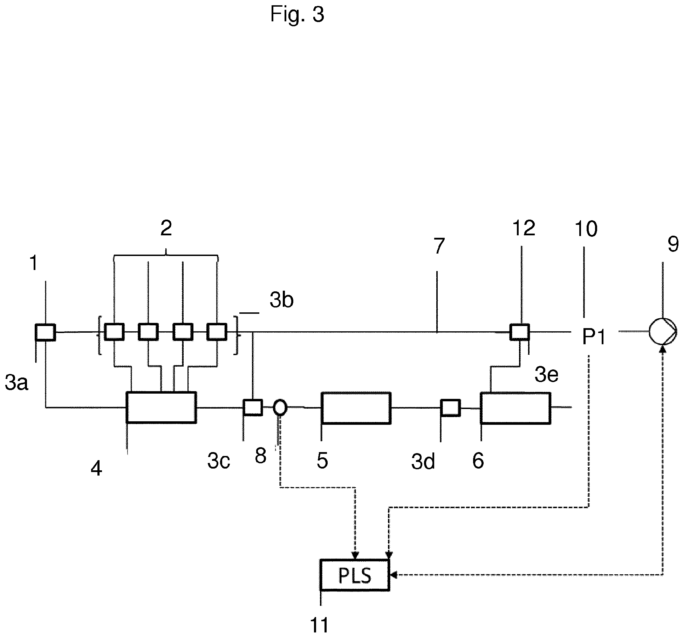

FIG. 3 shows a schematic drawing of how degassing and de-bubbling is carried out in a continuous pathogen-reduced method for the production of therapeutic proteins using a dense membrane

The objective during this task is to ensure a bubble free, as far as possible degassed, pathogen-reduced state at various locations.

The fluid stream comprising the product (14) is debubbled and degassed via a membrane module comprising a dense membrane (12a) and directed into a chromatographic device (15). The chromatographic buffers (16) are also debubbled and degassed via membrane modules comprising dense membranes (12b). The debubbling and degassing ensures that no gas bubbles influence the performance of the chromatographic device. The fluid stream leaving the chromatographic device flows via a membrane module comprising a dense membrane (12c) to the next unit operation, e.g. an ultrafiltration unit (18) with a recirculation line. Since gas bubbles can potentially damage the product in the fluid stream, e.g. via leading to precipitation of the product, a bubble sensor (17) for detection gas bubbles is employed at this location. The fluid stream leaving unit operation (18) is in turn debubbled and degassed via the membrane module comprising a dense membrane (12d) and flows into unit operation (19) e.g. a diafiltration unit. In addition the buffer (20) to be used in the diafiltration unit (19) has to be debubbled and gassed via the membrane modules comprising a dense membrane (12e). A vacuum is preferably provided via a central vacuum tubing (23) and a vacuum pump (24).The pressure on the vacuum side is measured using sensor (21). Said pressure can either be regulated locally or via the process control system (22). The process control system (PLS) (22) monitors alterations in sensor (17) i.e. the breakthrough of gas bubbles. Such a break through carries the potential risk of pathogens entering the fluid stream and/or the formation of gas bubbles, which can influence the performance of unit operations and/or damage the product comprised in the fluid stream. Thus, should a potential break through be detected by the process control system, the system can cause single unit operations or the whole production process to pause to that defective parts may be replaced.

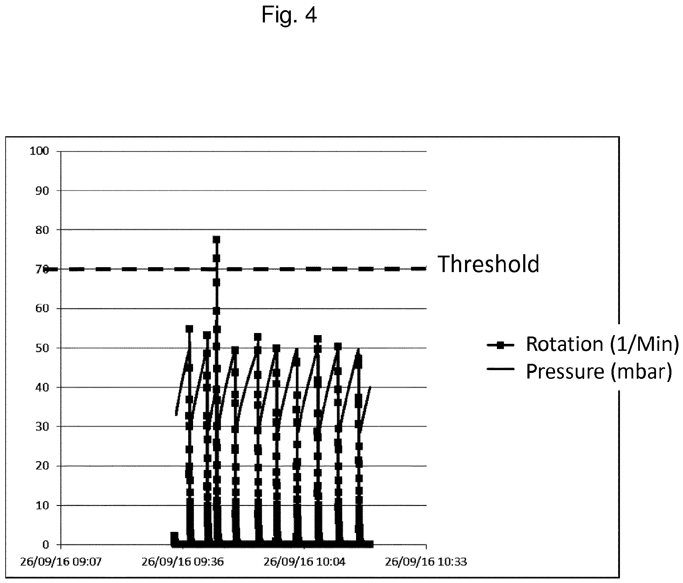

FIG 4 schematically depicts the control behavior of the vacuum pump in normal operation mode and the increase in rotational speed in the case of a leakage, which leads to the process steps being put on hold if the values increase above a predetermined threshold.

### EXAMPLES

### Example 1

In this example the module G681W of 3M was used as dense membrane. To test the maximum degassing rate the module was connected via an empty 3.2 mm cflex tubing to a storage vessel containing water. The exit of the module was sealed and the vacuum exit was connected to a vacuum pump with a pressure of 25 mbar. The maximum degassing rate of the empty module was 0,5 ml/min. It the fluid, i.e. the water, passed the module from bottom to top. At a pump rate of 20 ml/min even sporadic gas bubbles could not be separated from the fluid stream at the entrance of the module.

### Example 2

In a continuous pathogen-reduced method for the production of monoclonal antibodies and the system used for this production, respectively, dense membranes, in this case modules G681 W of 3M, were used. The modules were sterilized prior to installation via ethylenoxid treatment and were connected to the production system via aseptic connectors or welding. The flow rates of the fluid stream and the buffer solutions, respectively, varied between 0 - 30 ml/min. The flow into the G681W modules was via an 4.8 mm Cflex tubing. The flow direction was from top to bottom. The vacuum side of the module was connected via collection tubing with an inner diameter of 6mm with a vacuum pump. The employed vacuum pump was a Vacuubrand MD4CNT Vario with a vacuum controller CVC3000. The set point of the vacuum was 25 mbar and the point at which the pump started working was 50 mbar. Pressure and rotational speed of the pump were transmitted to a Siemens process control system PCS-7. If a threshold value was exceeded the rotational speed of process steps was put on hold.

The G681 W modules were installed at the following locations within the production process: The protein A chromatography was carried out using a BioSMB device from Pall. For this unit operation the G681W modules were installed directly prior to the suction side of the pumps. In this example, five G681 W modules were installed for the buffers and one for the incoming fluid stream. Moreover, two chromatography steps in flow-through mode, i.e. for polishing were also carried out on a BioSMB from Pall. For this unit operation the G681W modules were again installed directly prior to the suction side of the pumps. In this case three G681 W modules were installed for the buffers and two for the incoming fluid streams. In the subsequent unit operation - here a concentration step carried out in a continuous ultrafiltration in feed and bleed mode - a G681 W module was installed directly prior to suction side of the peristaltic pump for the feed flow. After continuous ultrafiltration the fluid stream was further processed in a continuous countercurrent diafiltration using a Gambro 2H dialysis module. In this unit operation a G681 W module was installed directly prior to the suction side of the peristaltic pump for the feed flow.

## Claims

1. Use of a dense membrane as pathogen barrier.

2. Use of at least one dense membrane and/or at least one nanoporous membrane in a method for the continuous, pathogen-reduced production and/or processing of a healthcare product.

3. Use according to claim 2, wherein the dense membrane and/or the nanoporous membrane is used as degasser and as pathogen barrier.

4. Use according to claims 2 and 3, wherein the dense membrane is gas-permeable but not air-permeable.

5. Use according to anyone of the preceding claims, wherein the dense membrane and/or the nanoporous membrane is situated in a fluid stream and the fluid stream is passed from top to bottom over the dense membrane.

6. Use according to claim 5, wherein the dense membrane and/or the nanoporous membrane is used as degasser and/or as de-bubbler and/or as pathogen barrier.

7. Use according to anyone of the preceding claims, wherein the dense membrane and/or the nanoporous membrane is operated using a vacuum.

8. Use according to claim 7, wherein the vacuum is generated using a vacuum pump and said pump is protected using a liquid trap.

9. Use according to anyone of the preceding claims, wherein the dense membrane and/or the nanoporous membrane is controlled by a process control system.

10. Use according to anyone of the preceding claims, wherein the dense membrane and/or the nanoporous membrane is situated in a fluid stream before said fluid stream enters a unit operation and/or passes a validation point selected from the group comprising an ultrafiltration unit for concentration, a recirculation loop, a unit for buffer or medium exchange preferably with concentration, e.g. an ultrafiltration, a bioburden reduction preferably with sterile filters, a capture chromatography , a virus inactivation, e.g. a coiled flow inverter i.e. a residence time module, a chromatographic intermediate and fine purification, e.g. ion exchange, mixed mode, hydrophobic interaction, SEC chromatography, a homogenization loop, a viral filtration, a flow cell for process analytics such as pH, conductivity, flow meter,a sample port for in process samples.

11. Method for the continuous, pathogen-reduced, modular production and/or processing of a healthcare product, wherein at least one dense membrane and/or at least one nanoporous membrane is employed for degassing and/or debubbling a fluid stream, wherein the fluid stream is passed top to bottom over the dense membrane.

12. Unit operation for the continuous, pathogen-reduced, modular production and/or processing of a healthcare product comprising at least one dense membrane and/or at least one at least one nanoporous membrane.
